Europäisches Patentamt

European Patent Office

Office européen des brevets

(11) Publication number: **0 297 455 B1**

(12) **EUROPEAN PATENT SPECIFICATION**

(45) Date of publication of patent specification: **17.06.92**   (51) Int. Cl.⁵: **A61K 7/06**

(21) Application number: **88110104.2**

(22) Date of filing: **24.06.88**

(54) **Compositions having hair stimulating activity.**

(30) Priority: **26.06.87 IT 2106387**

(43) Date of publication of application:
**04.01.89 Bulletin 89/01**

(45) Publication of the grant of the patent:
**17.06.92 Bulletin 92/25**

(84) Designated Contracting States:
**AT BE CH DE ES FR GB GR IT LI LU NL SE**

(56) References cited:
**EP-A- 0 182 756**
**FR-A- 2 036 453**
**FR-A- 2 591 889**
**GB-A- 2 167 958**

(73) Proprietor: **Crinos Industria Farmacobiologica S.p.A.**
**Piazza XX Settembre, n. 2**
**I-22079 Villa Guardia Como(IT)**

(72) Inventor: **Gazzani, Giovanni**
**Via Volta 28/B**
**I-22070 Appiano Gentile (Como)(IT)**

(74) Representative: **Dragotti, Gianfranco et al**
**SAIC BREVETTI s.a.s. Viale Bianca Maria, 15**
**I-20122 Milano(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to compositions with hair stimulating activity comprising as the active principle a mixture essentially containing sulfomucopolysaccharides (or glycosaminoglycanes or mucopolysaccharides), in which these components are present in determined amounts and determined weight ratios.

The use of these substances in cosmetic field is by the way known and widely documented since several years.

Thus, for example, FR-A- No. 2,036,453 (Henry), besides a method for the separation of glycosaminoglycane mixtures from other substances also coming from the alkaline extraction of animal organs, teaches the use of these mixtures as active principle in cosmetic compositions such as, for example, shampoo, soap, tooth pastes.

The organs from which said substances are extracted are selected among the following:

connective tissues (mucosae and skin), glands, viscera, embrions and umbilical cords.

In this connection it is to be noticed that the afore process is different from that by which the mixtures of the present invention are obtained, the latter being by the way already known in the art and being hereinafter reported as well as detailedly disclosed in the example 1. As a matter of fact, by adopting the method of the above cited French Patent, namely by selectively precipitating the sulfomucopolysaccharides by means of the corresponding salt with quaternay ammonium cations in a medium made saline by means of 0.5M NaCl, and subsequently separating said polymers from these complex compounds by means of a 1M $MgCl_2$ , and lastly precipitating again with quaternary cations in a 0.3 M $MgCl_2$ solution, a mixture of glycosaminoglycanes is obtained as the final result having a composition highly different from that of the present invention wherein moreover the heparan sulfate is present in a very reduced amount. This fact has been demonstrated by the Applicant by repeating the fractionating scheme used by Henry. This experiment is described in the following example 2.

On the product which has been obtained analyses have been carried out to determine the single amounts of glycosaminoglycanes as well as the residual impurities (proteins, nucleic acids).

On the basis of the results which have been obtained it has been thus concluded that in this patent, besides the fact that no composition of sulfomucopolysaccharides is reported corresponding to the extracts which are obtained with the used method, the same process would not be anyhow adapted to produce a mixture having qualitatively and quantitatively identical features as the composition of the present invention.

Furthermore it is to be noted that in the above mentioned patent the use of sulfomucopolysaccharides is not foreseen in any case as an active principle to promote the hair regrowth.

More recently the published European Patent Application No. 0182756 (Farmaka) discloses the use, as the active principle for cosmetic use, particularly with respect to the care of the baldness, of an aqueous extract of umbilical cord in combination with an essentially sulfomucopolysaccharidic mixture, with high content of condroitin sulfates and heparin like substances, the analytical profile of which is reported as regards both the chemical parameters (hexosamines, uronic acids, total sulfur and nitrogen, inorganic phosphor, proteins, specific rotation) and the biological ones, in terms of anticoagulating activity.

In this case too the disclosure of the invention is not able in any way to suggest the percentage ranges and the respective weight ratios between the sulfomucopolysaccharides present in the mixture of the present invention, with respect to the improvement of the hair stimulating activity which has been achieved.

Moreover from the prior art, as for instance in the UK Patent Application No. 2167958, it is evident that the use of the above mentioned substances as active principle in the hair stimulation was per se generically known and consequently in this field, before the above indicated Farmaka application, the efficacy of these mixtures containing glycosaminoglicanes in this particular field of the cosmesis was already made evident.

Coming now back to the content of the above European Patent Application, it is to be noted that for most of the analytical parameters which are taken into consideration only lower limits (hexosamines, uronic acids, sulfur) or upper limits (nitrogen) of variation, are indicated whereby it seems justified to state that owing to their genericity said limits might be referred to also to the single sulfomucopolysaccharides (in this connection see M.B. Mathews, J.A. Cifonelli "Acid Mucopolysaccharides, reference standards" characterization under contract No. N01-AM-5-2205 from the National Institute of Health, J.S. Brimacombe, J.M. Webber "Mucopolysaccharides "Elsevier Publishing Company 1964).

The only conclusions which can be drawn, from the whole of the analytical parameters referred to in the above European Patent Application, relate to the presence in the glycosaminoglycane mixture of greater amounts of heparin as it can be seen from the positive value of the specific rotation (see above references and from the fact that the subject extract has remarkable anticoagulating activity which, on the basis of the what was already known on the property in this respect of the other glycosaminoglycanes (A.N. Teien et

2

Alii: "The anticoagulant effect of heparan sulfate and dermatan sulfate" Thrombosis Res. 8 859, 1976), can not be integrally attributed to the heparin).

It is moreover remarkable the fact the there is no mention at all neither of the organs from which the mixture is extracted nor about the method uses for the isolation thereof.

All the above comments originating, as it has been seen both from a logical interpretation of the above analitycal data as well as from the consideration of the evident insufficiency of the description, can only add to the inadequacy of the teaching which can be desumed from the above invention as regards the definition of the composition of the single polymers of the mucopolysaccharidic mixture which is herein described.

Lastly, the UK Patent Application GB-A- 2167958 relates to a cosmetic composition containing 1 to 10% of extract of animal placenta together with 2 to 20% of a mixture of the following substances:
- 10 to 30% mucopolysaccharides;
- 20 to 30% proteolisat of bacterial cells;
- 2 to 10% peptides from calf serum;
- 20 to 30% peptides of embryonic skin.

(The above mentioned percentage are indicated by weight).

In this connection it is to be observed that even in that case no details are given about the extraction method used, which as it will be seen later on in the examples 1 and 2, may lead to results which are highly different in terms both of qualitative composition and of quantitative composition of the extract. As a conclusion, none of the above mentioned references might suggest in any way the fact that an essentially sulfomucopolysaccharidic composition, with the properties which shall be pointed out later on could have a hair stimulating activity greater than that of other mixtures, which although having the same qualitative composition, comprise said polymers in different weight amounts and ratios.

In the course of a more wide study about the hair stimulating activity of extracts from animal organs of the bovine or swine species at the applicant laboratories experiments have been carried out with extracts prepared so as to prevailingly contain glycosaminoglycanes and a very reduced amount of residual impurities (proteins and nucleic acids).

The extraction methods which have been used to obtain said mixtures are known in the art and consist in the alkaline extraction of the said organs followed by a filtration with a coadjuvant agent (for example Clarcel), subsequent enzimatic treatment and precipitation of the raw extract by addition of quaternary ammonium salt (J.E. Scott, Method of Biochemical Analysis, vol. 8 Pag. 147-1960).

The separation of the nucleic acid from the glycosaminoglycanes is carried out by selective precipitation of the corresponding zinc salt as it is described in the US Patent No. 3,770,720, by which a pratically complete removal of these substances is ensured.

The zinc salts of the sulfomucopolysaccharides are thereafter precipitated by addition of a non solvent agent (acetone or alcohol), dissolved again in alkaline solution and precipitated in the presence of 0.05M NaCl with quaternary ammonium salts, which are then decomposed to give the corresponding sodium salt.

It is by the way known to the skilled in the art man that the removal of the nucleic acids can be achieved also by precipitation of the corresponding calcium salt in acidic medium.

It is also not to be neglected the possibility of removing those polymers by attack with the suitable enzymes in the case that the amounts of said nucleic acids present are not relevant.

The organs to which the above method has been applied are the following:
- small gut, skin, lung, placenta, trachea and eyes.

Starting from 25 Kg of fresh organ, the percentage recoveries obtained have been the following ones:
- small gut : 0.06
- skin: 0.04
- lung : 0.05
- placenta: 0.02
- trachea: 0.04
- eyes: 0.01

In order to evaluate the hair stimulating activity the extracts have been dissolved in phyisiological solution at 1% concentration and injected by subcutaneous route (0.1 ml) in the back of Burgundy reddish rabbits (3), animals the back having being previously shaved.

The injections were carried out at two different heights and for each level in two symmetrical points with respect to the backbone of the animal.

Also two injections of physiological solution were carried out with the same above mentioned measures in order to get for each animal references by which the importance of the fenomenon being studied might be immediately evaluated.

The injections were repeated for 5 consecutive days. 7 days from the first injection and subsequently

3

after 15 and 30 days the hair regrowth was obversed.

The evaluation of the results was carried out by giving the following score of the basis of a visive appreciation of the regrowth:

- no regrowth

+ - just evident regrowth

+ evident regrowth

+ + good regrowth

+ + + high regrowth.

The average of the scores for each animal was carried out among the four injected areas and then a general average was calculated within each group of animal.

As regards the qualitative and quantitative analysis of the glycosaminoglycanes present in the extractive mixtures, the identification and the quantitative determination were carried out by electrophoresis according to B. Casu et Alii, Pharm. Res. Comm. 11, 297 1979; B. Casu et Alii, Arzneim. Forsch. 33, 1 135 1983.

If necessary suitable technics, more particularly specific enzymolisis, were used if during the quantitative analysis with the above mentioned method it was unavoidable to eliminate the interference of a mucopolysaccharide, the corresponding electrophoretic band of which would appear superimposed to that of the polymer being analized.

As regards the impurities, the amount of residual nucleic acids has been determined by assessing the phosphor according to Fiske-Subarrow, J. Biol. Chem. 66, 375 1925, assuming an average percent content of phosphor in said substances of 8.7%.

The total proteins were assessed as the sum of the single weight amounts of each aminoacid, the latter being in turn determined by an automatic analyzer of aminoacid.

At that point it is worth to observe that the above mentioned impurities have never been higher in the extracts which have been tested and which are hereinafter reported, than the amount of 0.5% for the nucleic acids and of 5% for the proteins, respectively.

In the following table 1, the results are reported of the experiments relating to the hair stimulating activity as previously mentioned together with quantitative composition expressed in sulfomucopolysaccharides of the single extract.

The abbreviations appearing in this table refer respectively to the following sulfomucopolysaccharides:

HP = Heparin

HS = Heparan sulphate

DS = Dermatan sulphate

CHSAC = Mixture of condroitin sulphates A and C

KS = Keratan sulphate

HA = Ialuronic acid

From the same table I it can be observed that the hair stimulating activity increases for those extracts in which some glycosaminoglycanes are simultaneously present, such as heparin, heparan sulphate, condroitins A and C, and dermatan sulphate as well as greater amounts of some of them exist, as it occurs in the case of trachea, wherein anyhow, differently from the skin, the condroitin are present in prevailing amount with respect to the dermatan sulphate.

From the studies carried out by the applicant it is remarkably observed that the extracts containing as main component ialuronic acid have a hair stimulating activity lower than the previous above mentioned extract. It has been found and is the object of the present invention that by providing mixtures of said extracts (for the binary mixtures reported in Table 2 the extracts are in the ratio 1:1) so as to obtain a final composition of glycosaminoglycane in which the single sulphomucopolysaccharides are present in weight percentage within determined variation ranges and moreover some of them are in determined weight ratios to each other, it is possible to furthermore improve the above mentioned hair stimulating activity with respect to that of the starting extracts.

In that way, thus it is possible to obtain active principles consisting of sulfomucopolysaccharidic extracts, having properties as later explained, and more active as regards the hair stimulation with respect to other extracts containing the same substances in weight percentage different from that which are taught in the present invention, and furthermore characterized in that said glycosaminoglycanes have weight ratios to each other different from those foreseen in the present application.

The results of the above tests are reported in table II.

From this table it can be desumed that in order to obtain the aforesaid synergic action the extract must contain in determined percentages and simultaneously heparin, heparan sulphate, condroitin sulphates A and C and dermatan sulphate, besides ialuronic acid and keratan sulfate as possible additional components.

Moreover as it can be observed from the following table III the most important polymers among those above referred to, must be present in the mixture in determined reciprocal weight ratios.

The analysis of the data of table II permits the following composition ranges, in weight percent, to be established, with respect to each mucopolysaccharide in the mixture having improved hair stimulating activity.

| Glycosaminoglycane | Main range | Preferred range |
|---|---|---|
| Heparin | 2-30 | 5-20 |
| Heparan sulphate | 10-40 | 13-35 |
| Dermatan sulphate | 10-35 | 20-32 |
| Condroitin sulphate A and C | 15-40 | 24-36 |
| Keratan sulphate | 0-10 | 0-5 |
| Ialuronic acid | 0-17 | 0-13 |
| Nucleic acid ≤0.5% , Protein ≦ 5% | | |

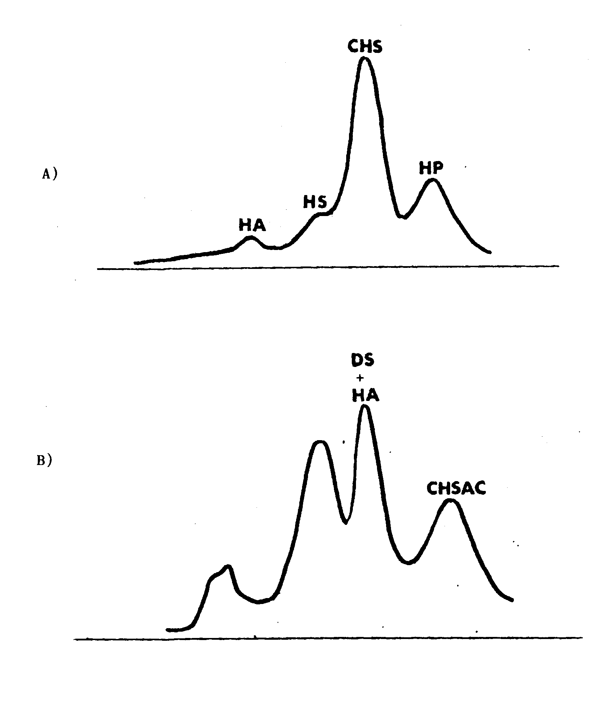

An example of electrophoretic profile of a mixture of sulfomucopolisaccharides according to the present invention and of the related quantitative dosage, obtained with the already cited methods, is reported in figure 1 in which the graph A relates to the HCl electrophoresis whereas the graph B relates to electrophoresis carried out in barium acetate buffer.

From this analysis the following composition results:

HP 12%

HS 16%

CHS-AC 28%

DS 30%

HA 11%

## TABLE I

Sulfomucopolysaccharidic mixtures, originating organ, composition and hair stimulating activity

| Organs* | Composition as glycosaminoglycanes** | Hair regrowth in the rabbit observing time | | |
|---|---|---|---|---|
| | | 7 days | 15 days | 30 days |
| Small gut (A) | HP 17%, HS 60%, CHSAC 6%, DS 12%, HA 4% | +− | ++ | ++ |
| Lung (B) | HP 13%, HS 27%, CHSAC 47%, DS 11% | +− | ++ | ++ |
| Placenta (C) | HP 5%, HS 27%, CHSAC 19%, DS 10%, HA 36% | +− | ++ | ++ |
| Skin (D) | CHSAC 24%, DS 50%, HA 24% | − | +− | + |
| Trachea (E) (cartilagin) | CHSAC 68%, DS 10%, HA 2%, KS 17% | + | + | ++ |
| Eyes (F) | CHSAC 9%, HA 80%, KS 5% | +− | +− | +− |

* the organs are indicated with a letter of the alphabet which is then used instead of the organ name in the following table II.

** the balance to 100 is due to the residual content of proteins.

EP 0 297 455 B1

If the mixtures of the previous tables I and II and which have shown an regrowth activity from good (++) to high degree (+++) respectively, are taken into considerations and if the related weight ratios of heparan sulphate, condroitin sulphate A and C, and dermatan sulphate with respect to the amount of heparin present are calculated, the results reported in table III are obtained.

## TABLE II

Extract obtained by mixing those of the previous Table I in the ratio 1:1, composition and hair stimulating activity.

| Mixture | Composition as glycosaminoglycanes ** | Hair regrowth in the rabbit obverving time | | |
| --- | --- | --- | --- | --- |
| | | 7 days | 15 days | 30 days |
| A+C | HP 10%, HS 45%, CHSAC 12%,  DS 10%, HA 21% | +− | + | ++ |
| A+D | HP 11%, HS 29%, CHSAC 16%,  DS 30%, HA 13% | + | ++ | +++ |
| A+E | HP 8%, HS 31%, CHSAC 36%, DS 12%, HA 3%, KS 8% | + | ++ | +++ |
| A+F | HP 8%, HS 29%, CHSAC 3%, DS 6%, HA 42%, KS 3% | + | ++ | ++ |
| B+D | HP 6%, HS 14%, CHSAC 35%, DS 31%, HA 12% | + | ++ | +++ |
| B+F | HP 6%, HS13%, CHSAC 29% DS 5%, HA 41%, KS 3% | +− | + | ++ |

**for the percentage what stated in the previous table holds true.

EP 0 297 455 B1

EP 0 297 455 B1

TABLE III

Weight ratios, referred to the amount of heparin present in the mixture of heparan sulphate condroitin sulphate A and C and dermatan sulphate in extracts having good (++) and high degree (+++) hair stimulating activity

|  | HP* | HS* | CHSAC* | DS |
|---|---|---|---|---|
| – Extracts with activity++ | | | | |
| A* (table I) | 1 | 3.5 | 0.3 | 0.7 |
| B* (table I) | 1 | 2 | 3.6 | 0.7 |
| C* (table I) | 1 | 5.4 | 3.8 | 2 |
| A*+C* (table II) | 1 | 4.5 | 1.2 | 1 |
| A*+F* (table II) | 1 | 3.6 | 0.4 | 0.7 |
| B*+F* (table II) | 1 | 2.2 | 4.8 | 0.8 |
| – Extracts with activity +++ | | | | |
| A*+D* (table II) | 1 | 2.6 | 1.4 | 2.7 |
| A*+E* (table II) | 1 | 3.9 | 4.5 | 1.5 |
| B*+D* (table II) | 1 | 2.3 | 5.8 | 5,2 |

* abbreviations as in the previous tables.

From the previous table it is seen that the sulfomucopolisaccharide mixture having improved hair stimulating activity is characterized by having besides determined composition ranges of the single glycosaminoglycanes, also determined ranges of weight ratios to each other , which referred to the amount of heparin are defined as follows: from 1 (HP): 2.3 (HS): 1,4 (CHSAC) : 1.5 (DS) to 1 (HP): 3.9 (HS): 5.8 (CHSAC) : 5.2 (DS).

As it is well known to the skilled in the art man the sulfomucopolisaccharide mixtures according to the present invention can be also obtained by extracting them directly from organs or organ mixtures, which are admixed in suitable proportions on the basis of the respective extraction yields of the single organs and with respect to the composition of glycosaminoglycanes which is sought for.

It is also evident that in a simpler manner as it has been carried out by the applicant , the above mixture can also be obtained by admixing in determined weight ratios two or even more extracts, so that the required composition is obtained.

The compositions according to the present invention can be formulated with the excipients or vehicles commonly used in cosmetic field, in form of lotion, gel, cream, ointment, etc.

Formulation examples are reported in the examples 3 and 4.

EXAMPLE 1

25 kg of small gut are finely ground in a mincing machine and suspended in 25 litres of water. After heating to 70°C 1.46 litres of 10 NaOH are added. The extraction is carried out for 12 hours as a whole. At the end the suspension is cooled, neutralized with concentrated HCl up to pH 6. An equal weight of filtration adjuvant (Clarcel) is added and the suspension is filtered on filter press. Then the filter is washed with 3 portion each of 5 litres, and then the filtrate is subjected the enzimolysis with papain at a temperature of 45°C, with the addition of a bacterial growth inhibiting agent, for 24 hours.

The enzyme is inactivated by heating to 90°C and then the precipitation takes place by adding 2 volumes of acetone.

225 g of products are recovered.

8

110 g are dissolved in 2 litres of distilled water.

Under stirring 200 ml of a solution comprising 100 g/l of $ZnCl_2$ and 200 g of filtration adjuvant are added and the filter cake is washed on the filter with several portions of warm deionized water. The zinc salt of sulfomucopolysaccharides is precipitated with 2 volumes of acetone. The resulting powder is dissolved in water, until a concentration of 0.5% is obtained , the pH is brough to definitely alkaline values by adding solid NaOH and NaCl is added up to a molarity of 0.05 M (the ionic force being controlled by conductinmetric tests) and then a solution of quaternary ammonium salts is added.

The precipitate is treated as detailedly referred in the above mentioned reference (J.E. Scott, Methods in Biochem. Vol. 8 pag. 147). There are obtained 10 g of a mixture the analytic characteristics of which are reported in table I as regards the quantitative composition of glycosaminoglycanes and moreover having a content of residual proteins of 0.08% and of nucleic acids of 0.03%.

EXAMPLE 2

Selective precipitation of mucopolysaccharides with quaternary ammonium salt in 0.05M NaCl saline solution (example B, pag. 4, of French Patent No. 2,036,453).

110 g of raw product obtained in the preceding example from the precipitation with acetone of the solution obtained from the extraction are dissolved in 7 litres of deionized water. An amount of NaCl sufficient to obtain a molarity of the salt of 0.5M is added.

Then 75 ml of a 10% solution of 0.5 M acetyl piridinium chloride in sodium chloride are added. The mixture is maintained at rest for 12 hours at room temperature , the filtration adjuvant is added and the mixture is filtered. The moist cake is suspended again in 2 litres of 1 $MgCl_2$ solution at 40°C and the mixture is filtered. Then the filtrate is added with a 0.05% solution of cetyl piridimum chloride (on the whole 4.7 litres so as to reduce the molarity of the solution from 1 to 0.3 M.)

After decomposition of the complex salt of quaternary ammonium, there are obtained 4 g of a product having the following quantitative composition. HP 49%, DS 36%, CHSAC 9%, HS 3%, residual proteins 2.7% , nucleic acids 0.3%.

EXAMPLE 3

Lotion

| | |
|---|---|
| Ethyl alcohol | ml 15 |
| propylene glycol | g 1.5 |
| preservant and perfume | g 0.8 |

| | |
|---|---|
| NTS* | g 0.5–5 |
| water | balance to ml 100 |

EXAMPLE 4

Gel

| | |
|---|---|
| Carbopol | g 1 |
| neutralizer | enough |
| NTS* | 0.5–5 |
| preservants | enough |
| water | balance to g 100 |

EXAMPLE 5

Cream

| | |
|---|---|
| Emulgade 100 NI | g 3 |
| NTS* | g 0.5–5 |
| preservants | enough |
| water | balance to g 100 |

* abbreviation of glycosaminoglycane mixture having the characteristics described in the present invention.

## Claims

1. Composition with hair stimulating activity, characterized by comprising, besides the normal excipients and vehicles for cosmetic use, an active ingredient consisting of a mixture of sulfomucopolysaccharides, having the following composition:

| | |
|---|---|
| heparin | 2-30% by weight |
| heparin sulphate | 10-40% by weight |
| dermatan sulphate | 10-35% by weight |
| chondroitin sulphate A + C | 15-40% by weight |
| keratan sulphate | 0-10% by weight |
| ialuronic acid | 0-17% by weight |
| nucleic acids | ≤ 0.5% by weigth |
| proteins | ≤ 5% by weight |

2. Composition with hair stimulating activity according to claim 1, characterized in that said active ingredient has the following composition

10

| heparin | 5-20% by weight |
| --- | --- |
| heparin sulphate | 13-35% by weight |
| dermatan sulphate | 20-32% by weight |
| chondroitin sulphate A + C | 24-36% by weight |
| keratan sulphate | 0-5% by weight |
| ialuronic acid | 0-13% by weight |
| nucleic acids | ≤0.5% by weigth |
| proteins | ≤5% by weight |

3. Composition with hair stimulating activity according to claim 1, characterized in that that the composition of said active ingredient, expressed as weight ratios between the single glycosaminoglycanes referred to the heparin amount , namely HP:HS:CHSAC:DS, is of between 1:2.3:1.4:1.5 and 1:3.9:5.8:5.2.

4. Composition with hair stimulating activity according to claim 1, characterized by containing as the active ingredient the extract of at least one organ selected among small gut, lung, placenta, said extract having an essentially reduced content of nucleic acids and of proteins.

5. Composition with hair stimulating activity according to claim 4, characterized by containing as the active ingredient, the mixture of at least two extracts, the first of which is selected among the extracts of small gut, lung and placenta, whereas the second extract is selected among those of skin, trachea and eyes.

6. Composition with hair stimulating activity according to claim 5, characterized in that said two extracts are respectively extracts of small gut and skin.

7. Composition with hair stimulating activity according to claim 5, characterized in that said two extracts are respectively extracts of small gut and trachea.

8. Composition with hair stimulating activity according to claim 5, characterized in that said two extracts are respectively extracts of lung and skin.

9. Composition with hair stimulating activity according to claim 3, characterized in that said components are mixed after having being independently prepared.

**Revendications**

1. Composition ayant une activité stimulante pour les cheveux, caractérisée en ce qu'elle comprend, en plus de excipients et véhicules normaux pour les applications cosmétiques, un ingrédient actif comprenant un mélange de sulfomucoplysaccharides, ayant la composition suivante :

| héparine | 2 à 30 % en poids |
| --- | --- |
| sulfate d'héparine | 10 à 40 % en poids |
| sulfate de dermatane | 10 à 35 % en poids |
| sulfate de chondroïtine A + C | 15 a 40 % en poids |
| sulfate de kératane | 0 à 10 % en poids |
| acide ialuronique | 0 à 17 % en poids |
| acides nucléiques | ≤ 0,5 % en poids |
| protéines | ≤ 5 % en poids |

2. Composition ayant une activité stimulante pour les cheveux selon la revendication 1, caractérisée en ce que ledit ingrédient actif a la composition suivante :

| héparine | 5 à 20 % en poids |
|---|---|
| sulfate d'héparine | 13 à 35 % en poids |
| sulfate de dermatane | 20 à 32 % en poids |
| sulfate de chondroïtine A + C | 24 à 36 % en poids |
| sulfate de kératane | 0 à 5 % en poids |
| acide ialuronique | 0 à 13 % en poids |
| acides nucléiques | ≦ 0,5 % en poids |
| protéines | ≦ 5 % en poids |

3. Composition ayant une activité stimulante pour les cheveux selon la revendication 1, caractérisée en ce que la composition dudit ingrédient actif, exprimée en proportions en poids entre les glycosaminoglycanes individuels et la quantité d'héparine, c'est-à-dire HP:HS:CHSAC:DS est comprise entre 1:2,3:1,4:1,5 et 1:3,9:5,8:5,2.

4. Composition ayant une activité stimulante pour les cheveux selon la revendication 1, caractérisée en ce qu'elle contient, comme ingrédient, actif, l'extrait d'au moins un organe choisi parmi, l'intestin grêle, le poumon, le placenta, ledit extrait ayant une teneur très réduite en acides nucléiques et en protéines.

5. Composition ayant une activité stimulante pour les cheveux selon la revendication 4, caractérisée en ce qu'elle contient, comme ingrédient actif, le mélange d'au moins deux extrait dont le premier est choisi parmi les extraits d'intestin grêle, de poumon et de placenta, tandis que le second extrait est choisi parmi ceux de peau, de trachée artère et d'yeux.

6. Composition ayant une activité stimulante pour les cheveux selon la revendication 5, caractérisée en ce que lesdits deux extraits sont respectivement des extraits d'intestin grêle et de peau.

7. Composition ayant une activité stimulante pour les cheveux selon la revendication 5, caractérisée en ce que lesdits deux extraits sont respectivement des extraits d'intestin grêle et de trachée-artère.

8. Composition ayant une activité stimulante pour les cheveux selon la revendication 5, caractérisée en ce que lesdits deux extraits sont respectivement des extraits de poumon et de peau.

9. Composition ayant une activité stimulante pour les cheveux, selon la revendication 3, caractérisée en ce que lesdits composants sont mélangés après avoir été préparés indépendamment.

**Patentansprüche**

1. Zusammensetzung mit anregender Wirkung für Haare, dadurch **gekennzeichnet,** dass sie neben den normalen Exzipienzien und Vehikeln für die kosmetische Verwendung einen aktiven Bestandteil, bestehend aus einer Mischung aus Sulfomucopolysacchariden der folgenden Zusammensetzung enthält;

| Heparin | 2 bis 30 Gew.% |
|---|---|
| Heparinsulfat | 10 bis 40 Gew.% |
| Dermatansulfat | 10 bis 35 Gew.% |
| Chondroitinsulfat A + C | 15 bis 40 Gew.% |
| Keratansulfat | 0 bis 10 Gew.% |
| Ialuronsäure | 0 bis 17 Gew.% |
| Nukleinsäuren | ≦ 0,5 Gew.% |
| Proteine | ≦ 5 Gew.% |

2. Zusammensetzung mit anregender Wirkung für Haare nach Anspruch 1, dadurch **gekennzeichnet,** dass der aktive Bestandteil die folgende Zusammensetzung hat:

| Heparin | 5 bis 20 Gew.% |
|---|---|
| Heparinsulfat | 13 bis 35 Gew.% |
| Dermatansulfat | 20 bis 32 Gew.% |
| Chondroitinsulfat A + C | 24 bis 36 Gew.% |
| Keratansulfat | 0 bis 5 Gew.% |
| Ialuronsäure | 0 bis 13 Gew.% |
| Nukleinsäuren | $\leq$ 0,5 Gew.% |
| Proteine | $\leq$ 5 Gew.% |

3. Zusammensetzung mit anregender Wirkung für Haare nach Anspruch 1, dadurch **gekennzeichnet,** dass die Zusammensetzung des aktiven Bestandteils, auzgedrückt als Gewichtsverhältnisse zwischen den einzelnen Glycosaminoglykanen in bezug zur Heparinmenge, nämlich HP:HS:CHSAC:DS, zwischen 1:2,3:1,4:1,5 und 1:3,9:5,8:5,2 liegt.

4. Zusammensetzung mit anregender Wirkung für Haare nach Anspruch 1, dadurch **gekennzeichnet,** dass sie als aktiven Bestandteil den Extrakt von mindestens einem Organ, ausgewählt aus Dünndarm, Lunge, Plazenta, enthält, wobei der Extrakt einen im wesentlichen reduzierten Gehalt an Nukleinsäuren und Proteinen aufweist.

5. Zusammensetzung mit anregender Wirkung für Haare nach Anspruch 4, dadurch **gekennzeichnet,** dass sie als aktiven Bestandteil eine Mischung aus mindestens zwei Extrakten enthält, wobei der erste Extrakt aus den Extrakten von Dünndarm, Lunge und Plazenta ausgewählt ist, wohingegen der zweite Extrakt aus denen von Haut, Trachea und Augen ausgewählt ist.

6. Zusammensetzung mit anregender Wirkung für Haare nach Anspruch 5, dadurch **gekennzeichnet,** dass die beiden Extrakte Extrakte von Dünndarm bzw. Haut sind.

7. Zusammensetzung mit anregender Wirkung für Haare nach Anspruch 5, dadurch **gekennzeichnet,** dass die beiden Extrakte Extrakte von Dünndarm bzw. Trachea sind.

8. Zusammensetzung mit anregender Wirkung für Haare nach Anspruch 5, dadurch **gekennzeichnet,** dass die beiden Extrakte Extrakte von Lunge bzw. Haut sind.

9. Zusammensetzung mit anregender Wirkung für Haare nach Anspruch 3, dadurch **gekennzeichnet,** dass die Komponenten gemischt werden, nachdem sie unabhängig voneinander hergestellt worden sind.

A)

B)